# EUROPÄISCHE PATENTSCHRIFT

(11) **EP 4 517 300 B1**
(45) Veröffentlichungstag und Bekanntmachung des Hinweises auf die Patenterteilung: **10.09.2025**
(21) Anmeldenummer: 23194577.5
(22) Anmeldetag: 31.08.2023
(51) Int. Cl.: B27N 1/02, B27N 3/02, B27N 3/14, B27N 3/18, G01N 21/3563, G01N 21/359, G01N 33/46, G01N 21/84

(54) **MESSVERFAHREN ZUR ONLINE BESTIMMUNG EINER AUF HOLZPARTIKEL AUFGETRAGENEN BINDEMITTELMENGE IN EINER PRODUKTIONSANLAGE ZUR HERSTELLUNG VON HOLZWERKSTOFFPLATTEN**
MEASURING METHOD FOR ONLINE DETERMINATION OF A BINDING AGENT AMOUNT APPLIED TO WOOD PARTICLES IN A PRODUCTION PLANT FOR PRODUCING WOOD MATERIAL BOARDS
PROCÉDÉ DE MESURE EN LIGNE D'UNE QUANTITÉ DE LIANT APPLIQUÉE SUR DES PARTICULES DE BOIS DANS UNE INSTALLATION DE PRODUCTION POUR LA FABRICATION DE PANNEAUX EN BOIS

(43) Veröffentlichungstag der Anmeldung: 05.03.2025
(73) Patentinhaber: Flooring Technologies Ltd., Kalkara SCM1001 (MT)
(72) Erfinder: ZHANG, Jinming, 10247 Berlin (DE); KALWA, Norbert, 32805 Horn-Bad Meinberg (DE)
(74) Vertreter: Maikowski & Ninnemann Patentanwälte Partnerschaft mbB

(56) Entgegenhaltungen:
- WO-A2-2007/108873
- US-A1- 2004 094 851
- US-A1- 2004 195 714
- US-A1- 2007 131 862

## Beschreibung

Die vorliegende Erfindung betriff ein Verfahren zur Bestimmung einer Bindemittelmenge aufgetragen auf Holzpartikel in einer Produktionsanlage zur Herstellung von Holzwerkstoffplatten, insbesondere von OSB-Platten.

### Beschreibung

Zur Herstellung von Holzwerkstoffplatten, wie OSB oder Spanplatten, werden Holzpartikel mit einem Bindemittel oder Leim versehen, die beleimten Holzpartikel auf ein Transportband gestreut und zu Platten verpresst. Als Bindemittel kommen häufig flüssige, meist wässrige Formulierungen, wie z.B. Melamin-Formaldehyd-Harz oder Harnstoff-Formaldehyd-Harz, zur Anwendung. Ein Beispiel für ein solches Verfahren ist im Patentdokument US 2004/094851 A1 angegeben. Nach der in US 2004/094851 A1 beschriebenen Methode werden zunächst die äußeren Schicht der OSB bildenden Holzstrands mit Phenolharz besprüht. Nachfolgend werden die Strands für die OSB-Kernlage aufgestreut. Die Strands für die OSB-Kernlage können mit anderen Harzen beladen sein als die äußeren OSB-Schichten.

Allerdings gibt es auch Leimsysteme, die kein Wasser enthalten. Zu diesen Leimsystemen zählt PMDI (polymeres Diphenylmethandiisocyanat). Dieses Leimsystem hat einige Vorteile gegenüber den klassischen wässrigen Leimsystemen z.B. auf Basis von Harnstoff-Formaldehyd. Der erste Vorteil ist, dass diese Leime keine Formaldehydemission zeigen. Der zweite Vorteil ist, dass man mit diesem Leim wasserbeständige Verleimungen/platten erzeugen kann und der dritte Vorteil ist, dass man relativ wenig Leim benötigt, um damit Platten herzustellen. Üblicherweise reichen 2 - 5 Gew% für die verschiedenen Holzwerkstoffe aus. Diese geringe Menge erzeugt aber ein Problem, da erhöhte Sorgfalt auf die gute Verteilung des Leimes gelegt werden muss. Dies gilt umso mehr je grösser die zu beleimenden Holzpartikel sind. So ist bei kleineren Holzspänen, wie Deckschichtspänen, für eine Spanplatte eine homogene Beleimung in z. B. einem Trogmischer deutlich leichter zu erreichen, als die Beleimung von größeren, flächigen Holzsstrands, wie Deckschichtstrands, für eine OSB (oriented strand board) in einem Coil (rotierende Trommel).

Dabei ist besonders problematisch, dass die Leimverteilung auf den Holzpartikeln nicht visuell beurteilt werden kann. Z.B. ist PMDI nur leicht bräunlich und kann deshalb z. B. auf gelben Strands aus Nadelholz nicht erkannt werden.

Zudem ist es bei auftretenden Problemen schwierig die Ursache zu ermitteln, da lediglich die Dosiermenge bekannt ist, aber nichts über die Verteilung des Leims. Versuche dieses Problem durch Zumischung von UV-aktiven Stoffen zu dem PMDI, zeigten zwar gewisse Erfolge, da man mit Hilfe von UV-Lampen die Leimverteilung sichtbar machen kann. Allerdings sind die Farbstoffe teuer und erhöhen somit die Kosten. Außerdem muss die Beurteilung trotzdem visuell vorgenommen werden und bleibt damit subjektiv. Eine kontinuierliche Überwachung ist mit diesem System ohnehin nicht möglich.

Der Einsatz von Bindemitteln wie PMDI von unterschiedlichen Herstellern mit unterschiedlichen Eigenschaften der Leime (Viskosität, Benetzung usw.) führt ebenfalls zu einer Verkomplizierung der Produktion. Auch die Verwendung von Zuschlagsstoffen (z. B. Flammschutzmittel) kann die Verteilung des Leimes signifikant verändern, ist aber nicht detektierbar. All dies führt dazu, dass wegen der leicht höheren Dosierung höhere Kosten entstehen und ein Mehrverbrauch an Rohstoff erfolgt.

Generell muss also festgestellt werden, dass an die Dosierung und die Verteilung des Leims auf den Holzpartikeln deutlich höhere Anforderungen gestellt werden. Weiterhin ist auch zu konstatieren, dass eine Fehlersuche deutlich schwieriger ist. Zwar können auftretende Probleme durch die an den Produktionsanlagen befindlichen Messsysteme (Spaltererkennung, Dickenmessung usw.) detektiert werden, jedoch sind damit die Ursachen nicht erkannt. Zudem muss erst eine Messvorrichtung eine Auffälligkeit zeigen, damit Maßnahmen eingeleitet werden. Dies bedeutet, dass unter Umständen größere Mengen an nicht qualitätsgerechter Ware entstehen.

Daraus ergeben sich verschiedene Nachteile, wie fehlende in-line Kontrolle, Nachjustierung mit Zeitverzug, Materialverluste oder nicht erkennbare Schwankungen in der Zusammensetzung.

Der Erfindung liegt daher die technische Aufgabe zu Grunde eine zerstörungsfreie Methode zu entwickeln, die die Auftragsmenge und insbesondere die Verteilung eines Bindemittels, wie PMDI, auf Holzpartikeln bestimmen kann. Die Methode soll in-line in Produktionslinien installiert sein und möglichst viele Messungen pro Zeiteinheit in Fertigungsrichtung und quer dazu liefern. Durch die Messungen soll der Produktionsprozess nicht gestört werden. Auch sollen damit Unregelmäßigkeiten bezüglich des Leimauftrags und der Leimverteilung früher erkannt werden bzw. dies als Ursache für die Unregelmäßigkeiten ausgeschlossen werden können.

Diese Aufgabe wird erfindungsgemäß durch ein Verfahren mit den Merkmalen des Anspruchs 1 gelöst.

Entsprechend wird ein Messverfahren zur online Bestimmung einer auf Holzstrandsaufgetragenen Menge und Verteilung eines Isocyanat-haltigen Bindemittels in einer Produktionsanlage zur Herstellung von Holzwerkstoffplatten bereitgestellt, wobei das Verfahren die folgenden Schritte umfasst:
- Aufbringen von mindestens einem Isocyanathaltigen Bindemittel in jeweils unterschiedlichen quantitativ definierten Mengen auf jeweilige Proben aus Holzstrands als Referenzproben und Aufstreuen der Referenzproben auf ein Transportband der Produktionsanlage;
- Aufnahme von mindestens einem NIR-Spektrum jeder der auf das Transportband aufgestreuten Referenzproben unter Verwendung von mindestens einem NIR-Messkopf in einem Wellenlängenbereich zwischen 1800 (5555 cm-¹ ) und 2500 nm (4000 cm-¹);
- Zuordnung der unterschiedlichen quantitativen Mengen an Isocyanathaltigen Bindemittel der auf das Transportband aufgestreuten Referenzproben zu den aufgenommenen NIR-Spektren der genannten Referenzproben; und
- Erstellung eines Kalibriermodells für den Zusammenhang zwischen den spektralen Daten der NIR-Spektren und den dazugehörigen quantitativen Mengen an Isocyanathaltigen Bindemittel der auf das Transportband aufgestreuten Referenzproben mittels einer multivariaten Datenanalyse;
- Aufbringen von mindestens einem Isocyanathaltigen Bindemittel auf Holzstrands (als zu vermessende Proben) und Aufstreuen der mit dem Isocyanathaltigen Bindemittel versehenen Holzstrands auf ein Transportband,
- Aufnehmen von mindestens einem NIR-Spektrum der mit dem Isocyanathaltigen Bindemittel versehenen und auf das Transportband aufgestreuten Holzpstrands unter Verwendung des mindestens einen NIR-Messkopfes in einem Wellenlängenbereich zwischen 1800 (5555 cm-¹) und 2500 nm (4000 cm-¹);und
- Bestimmen der quantitativen Menge des Isocyanathaltigen Bindemittels durch Vergleich des für die mit dem Isocyanathaltigen Bindemittel versehenen Holzstrands aufgenommenen NIR-Spektrums mit dem erstellten Kalibriermodell,
wobei die Menge an auf die Holzstrands aufgetragenem Isocyanathaltigen Bindemittel zwischen 1,0 und 3,5 Gew% beträgt.

Das vorliegende Verfahren ermöglicht demnach die Bestimmung der auf Holzstrands als Holzpartikel aufgetragenen Bindemittelmenge in einer Produktionsanlage für Holzwerkstoffplatten, wobei die mit dem Bindemittel versehenen Holzpartikel auf ein Transportband aufgestreut vorliegen; d.h. das Bindemittel wird auf die Holzpartikel aufgebracht, insbesondere aufgesprüht, die beleimten Holzpartikel auf ein Transportband aufgestreut und unmittelbar nach dem Aufstreuen vermessen. Somit erfolgt die NIR-Messung direkt am Transportband in der Fertigungslinie nach dem Verlassen einer Vorrichtung zum Streuen der Holzpartikel.

Das vorliegende Verfahren erlaubt nicht nur eine Bestimmung der Auftragsmenge sondern auch eine Aussage über die Verteilung des Bindemittels, wie z.B. PMDI auf den Holzpartikeln. Wie beschrieben, werden zunächst NIR-Spektren von verschiedenen Auftragsmengen des Bindemittels auf Holzpartikeln erstellt. Als Holzpartikel können neben den Strands gemäß der Erfindung und Späne zur Anwendung kommen. Diese werden unter definierten Bedingungen mit dem Bindemittel z.B. PMDI besprüht. Anschließend werden NIR-Spektren von diesen Referenzproben aufgenommen. Danach werden Kalibrationsmodelle erstellt mit deren Hilfe dann auf unbekannte Mengen und Zusammensetzungen geschlossen werden kann. Ein weiterer Vorteil ist, dass aus den NIR-Spektren auch weitere Parameter (z. B.: Feuchte, Holzart usw.) analysiert werden können, was eine mögliche Problemanalyse erleichtert.

Zwar ist die Analyse von Mischungen aus Holzpartikeln und Bindemittel unter Verwendung von spektroskopischen Methoden bekannt, jedoch erfolgt die Vermessung in diesen Fällen jeweils zu anderen Zeitpunkten des Produktionsprozesses bzw. an Einzelproben.

So wird in der US 2003/0048440 A1 ein Verfahren zur kontinuierlichen Bestimmung von Eigenschaften von Holzfasern (wie Faserlängenverteilung, Harzgehalt im Strom der harzbeschichteten Fasern) in einem Holzfaserstrom zur Herstellung von Holzfaserplatten beschrieben. Das Verfahren verwendet NIR-Strahlung zur Bestimmung der verschiedenen Eigenschaften des Holzfaserstroms unmittelbar nach der Zerfaserung vor dem Aufstreuen der Fasern auf ein Transportband.

US 2007/131862A1 beschreibt die Verwendung von NIR-Spektroskopie zur quantitativen Analyse von Harz-Holzwerkstoff-Kompositen. Es werden gleichzeitig Konzentrationen von verschiedenen Komponenten in einer Probe eines Harz-Holzkomposits gemessen. Beispielsweise werden die Komponenten Harz, Wachs, Polyole, anorganische Härter mit OSB Strands vermischt und anschließend mittels NIR-Spektroskopie vermessen und Kalibrationsmodelle erstellt. Die Kalibrationsmodelle werden anschließend zur quantitativen Analyse von unbekannten Harz-Holzproben verwendet. Zwingende Voraussetzung für die Messung ist eine Zerkleinerung (Vermahlen) der zu analysierenden Proben außerhalb des Produktionsprozesses. Eine Beurteilung der Menge und Verteilung von auf Holzpartikel (wie z.B, Holzstrands) aufgetragenem Bindemittel auf einem Transportband in einer Produktionslinie, wie erfindungsgemäß gefordert, ist damit nicht möglich.

In einer Ausführungsform wird das vorliegende Verfahren in einer Produktionsanlage mit einer Anlagengeschwindigkeit zwischen 200 und 500 mm/sec, bevorzugt zwischen 300 und 400 mm/sec durchgeführt.

In einer weitergehenden Ausführungsform des vorliegenden Verfahrens ist vorgesehen, dass der mindestens eine NIR-Messkopf sich quer zur Laufrichtung des mit den beleimten Holzpartikeln bestreuten Transportbandes bewegt und über die gesamte Breite des Transportbandes traversiert.

Des Weiteren ist vorteilhafterweise vorgesehen, dass der mindestens eine NIR-Messkopf mehrere Spektren pro Minute, bevorzugt bis zu acht Spektren pro Minute oder mehr aufnimmt, aus denen ein Mittelwert über die Anzahl der aufgenommenen Spektren gebildet wird.

Auch sollte der verwendete NIR-Messkopf unempfindlich gegenüber Temperaturschwankungen, Staub und Emissionen von Holzinhaltstoffen.

Das vorliegende Verfahren ermöglicht die Bereitstellung der Messwerte in kurzer Zeit (online, bevorzugt ohne störende Zeitverzögerung). Die Messdaten können zur Qualitätssicherung, Forschung und Entwicklung, zur Prozesskontrolle, Prozessregelung, Prozesssteuerung usw. eingesetzt werden. Durch den Messvorgang wird die Produktionsgeschwindigkeit usw. nicht reduziert. Grundsätzlich wird damit die Überwachung der Produktion verbessert. Zudem werden auch Stillstandszeiten durch Qualitätsbestimmungen und Anlagenjustierungen reduziert.

Die mit dem vorliegenden Verfahren mögliche Bestimmung der Auftragsmenge an Bindemittel auf Holzpartikel erfolgt bevorzugt ausschließlich mittels NIR-Messung. Eine Kombination mit anderen spektroskopischen Methoden, insbesondere unter Verwendung von anderen Wellenlängen außerhalb des NIR-Bereiches ist nicht vorgesehen.

Erfindungsgemäß erfolgt somit der Einsatz eines NIR-Messkopfes, bevorzugt eines NIR-Multimesskopfes, der über die Aufnahme von spektralen Daten (Spektren) im nahinfraroten Bereich (1350-2500 nm) eine Bestimmung der Menge und Verteilung an auf Holzpartikel aufgebrachtes Bindemittel zulässt. Die NIR-Strahlung wechselwirkt mit den organischen Funktionsgruppen, wie zum Beispiel O-H, C-H und N-H, die im Bindemittel, wie z.B. PMDI vorhanden sind. Während der Wechselwirkung wird die NIR-Strahlung durch die gemessene Probe gestreut und reflektiert. Durch den Empfang der reflektierten NIR-Strahlung per NIR-Detektor wird ein NIR-Spektrum erzeugt. Bei dieser Messung werden in einer Sekunde eine Vielzahl von einzelnen NIR-Messungen durchgeführt, sodass auch eine statistische Absicherung der Werte gewährleistet wird. Die NIR-Spektroskopie zusammen mit der (unten angeführten) Multivariaten Datenanalyse bietet eine Möglichkeit an, einen direkten Bezug zwischen den spektralen Informationen (NIR-Spektren) und den zu bestimmenden Parametern des aufgebrachten Bindemittels herzustellen.

Das vorliegende Verfahren nutzt den Umstand aus, dass die NIR-Strahlung an der Oberfläche der Partikeloberfläche reflektiert bzw. gestreut wird. Die reflektierte bzw. gestreute NIR-Strahlung wird von dem NIR-Detektor erfasst, und das ermittelte NIR-Spektrum wird zur Bestimmung der gewünschten Parameter (hier Auftragsmenge an Bindemittel) verwendet.

Für die Bestimmung der aufgebrachten Menge an Bindemittel werden bevorzugt spektrale Daten aus dem gesamten aufgenommenen Spektralbereich verwendet, d.h. es wird nicht eine einzelne, diskrete Wellenlänge, sondern vielmehr ein ganzer Bereich von mehreren Wellenlängen verwendet.

Gemäß dem erfindungsgemäßen Verfahren werden demnach zunächst Referenzproben von mit Bindemittel versehenen Holzpartikeln bereitgestellt. Für die Bereitstellung der Referenzproben werden verschiedene Mengen an Bindemittel, z.B. 1,0 Gew%, 2,0 Gew%, 3 Gew%, 4 Gew%, 5 Gew% (bezogen auf die Menge an Holzpartikel) auf die Holzpartikel aufgebracht.

Zu beachten ist auch, dass die Referenzprobe gleichartig zu der zu vermessenden Probe ist; d.h. Bindemittel und Holzpartikel der Referenzprobe weisen die gleiche Zusammensetzung wie das zu vermessende Bindemittel und Holzpartikel auf. Die Gleichartigkeit von zu vermessender Probe und Referenzprobe ist insbesondere bei Verwendung von Bindemitteln mit Zusatzstoffen wie Flammschutzmitteln, Fasern, weiteren Additiven bedeutsam.

Von diesen Referenzproben wird erfindungsgemäß zumindest ein NIR-Spektrum in einem Wellenlängenbereich zwischen 1800 nm (5555 cm-¹ ) und 2500 nm (4000 cm-¹) aufgenommen. Es ist auch möglich einen Wellenlängenbereich zwischen 1350 nm (7407 cm⁻¹) und 1600 nm (6250 cm⁻¹) alleine oder in Kombination mit einem Wellenlängenbereich zwischen 1800 (5555 cm⁻¹) und 2500 nm (4000cm⁻¹) zu verwenden.

Die unterschiedlichen quantitativen Mengen an Bindemittel der Referenzproben werden dann den jeweils aufgenommen NIR-Spektren dieser Referenzproben zugeordnet und es wird ein Kalibriermodell für den Zusammenhang zwischen den spektralen Daten der NIR-Spektren der Referenzproben und den dazugehörigen Bindemittelmengen als Parameterwert mittels einer multivariaten Datenanalyse erstellt; d.h. zu jedem Parameterwert der Referenzprobe korrespondiert ein NIR-Spektrum der Referenzprobe. Die für die verschiedenen Parameter erstellten Kalibriermodelle werden in einem geeigneten Datenspeicher hinterlegt.

Anschließend wird mindestens eine Messprobe umfassend ein auf Holzpartikel aufgebrachtes Bindemittel bereitgestellt, und mindestens ein NIR-Spektrum des auf die Holzpartikel aufgetragenen Bindemittels aufgenommen. Die quantitative Menge des auf die Holzpartikel aufgebrachten Bindemittelmenge kann durch Vergleich des für die Messprobe aufgenommenen NIR-Spektrums mit dem erstellten Kalibriermodell ermittelt werden.

Ein Vergleich und die Interpretation der NIR-Spektren erfolgt sinnvollerweise über den gesamten aufgenommenen Spektralbereich. Dies wird vorteilhaft mit einer an sich bekannten multivariaten Datenanalyse (MDA) durchgeführt. Bei multivariaten Analysemethoden werden in an sich bekannter Weise typischerweise mehrere statistische Variablen zugleich untersucht. Hierzu wird bei diesen Methoden üblicherweise die in einem Datensatz enthaltene Zahl von Variablen reduziert, ohne gleichzeitig die darin enthaltene Information zu mindern.

Im vorliegenden Fall erfolgt die multivariate Datenanalyse über das Verfahren der Partial Least Squares Regression (partielle Regression der kleinsten Quadrate, PLS) wodurch ein geeignetes Kalibrationsmodell erstellt werden kann. Die Auswertung der gewonnenen Daten wird bevorzugt mit einer geeigneten Analysesoftware vorgenommen wie z.B. mit der Analysesoftware SIMCA-P der Firma Umetrics AB oder The Unscrambler der Firma CAMO.

In einer weiteren Ausführungsform ist vorgesehen, für die Erstellung des Kalibriermodells spektrale Daten aus dem NIR- Spektralbereich zwischen 1350 (7400 cm-¹ ) und 2500 nm (4000 cm-¹) zu verwenden, die mittels geeigneter mathematischer Methoden vorbehandelt werden und anschließend der multivariaten Datenanalyse zugeführt werden. Es ist auch möglich einen Wellenlängenbereich zwischen 1350 nm (7407 cm⁻¹) und 1600 nm (6250 cm⁻¹) alleine oder in Kombination mit einem Wellenlängenbereich zwischen 1800 (5555 cm⁻¹) und 2500 nm (4000cm⁻¹) zu verwenden.

Die Bedeutung einer Wellenlänge für die Vorhersage von Parametern, wie der Bindemittelmenge, aus dem NIR-Spektrum wird mit Hilfe der Regressionskoeffizienten dargestellt. Dabei haben die Regionen mit großen Koeffizientenbeträgen starken Einfluss auf das Regressionsmodell. So zeigt die Darstellung der Regressionskoeffizienten in einem PLS-Regressionsmodell für die Bestimmung der Bindemittelmenge, dass der Wellenlängenbereich zwischen 1800 nm (5555 cm-¹ ) und 2500 nm (4000 cm-¹) mit Maxima bei 1980 nm (5050 cm⁻¹) und 2500 nm (4000 cm-¹) für die Berechnung des Modells am wichtigsten ist, da hier die Beträge der Regressionskoeffizienten am größten sind. Die anderen Bereiche im Spektrum haben zwar geringeren Informationsgehalt in Bezug auf die NIR-Messung, tragen aber dennoch dazu bei, die weiteren Informationen bzw. störenden Einflussgrößen (wie Transparenz des Bindemittels, Oberflächenbeschaffenheit der Holzpartikel usw.) zu berücksichtigen bzw. zu minimieren.

Zur Eliminierung von störenden Einflüssen (wie z.B. Beschaffenheit der Oberfläche der Holzpartikel, Farbigkeit der Proben, Lichtstreuung an den Holzpartikeln oder anderen Additiven usw.) ist es notwendig, die spektralen Daten mit mathematischen Vorbehandlungsmethoden (z. B. derivative Datenvorbehandlung, Standardisierung gemäß SNVT (Standard Normal Variate Transformation), multiplikative Signal-Korrektur (EMSC, Extended Multiplicative Signal Correction usw.) zu bearbeiten. Dabei werden die Basislinieneffekte, die hauptsächlich durch die unterschiedliche Farbe der Proben verursacht werden, aus den Spektren entfernt, überlagernde Banden voneinander getrennt und die Abhängigkeit der Lichtstreuung an den Holzpartikeln berücksichtigt.

Wie oben bereits erwähnt, ermöglicht das vorliegende Verfahren nicht nur die Bestimmung der auf die Holzpartikel aufgebrachten Bindemittelmenge, sondern auch eine Aussage über die Verteilung des Bindemittels über bzw. zwischen den Holzpartikeln, die auf das Transportband aufgestreut sind.

So ist es möglich, durch einen Vergleich von tatsächlich den Holzpartikeln zugeführter Binde- bzw. Leimmittelmenge mit der am Transportband für die aufgestreuten, beleimten Holzpartikel gemessener Bindemittelmenge eine Aussage über die Verteilung des Bindemittels über die Holzpartikel zu treffen. Liegt eine schlechte Verteilung des Bindemittels vor, sinkt der Mittelwert des Messsignals ab und es wird ein durchschnittlich geringerer Bindemittelanteil gemessen als ursprünglich aufgetragen, was als Indiz für eine schlechte Bindemittelverteilung gesehen werden kann. So kann im Falle einer schlechten Verteilung des Bindemittels über die Holzpartikel die gemessene Bindemittelmenge bis zu 10-20 % von der tatsächlich zugeführten Bindemittelmenge abweichen.

Auch ist eine schlechte Verteilung des Bindemittels mit einer starken Schwankung der gemessenen Bindemittelmenge verbunden. So können bei einer schlechten Verteilung des Bindemittels die gemessenen Werte für das Bindemittel zwischen 30-40% um einen Mittelwert schwanken, während bei einer guten Verteilung des Bindemittels die gemessenen Werte für das Bindemittel zwischen 15-30% um einen Mittelwert schwanken.

Die Bestimmung der Verteilung des Bindemittels ist bedeutsam, da ein inhomogener Auftrag und somit eine inhomogene Verteilung des Bindemittels auf Holzpartikel, insbesondere bei größeren Holzpartikeln, wie Holzstrands, zu einer schwankenden Querzugfestigkeit der verpressten Holzwerkstoffplatten führen kann. Das vorliegende Verfahren eröffnet die Möglichkeit, die mittels NIR-Spektroskopie bestimmte Bindemittelmenge und Bindemittelverteilung in Bezug zu technologischen Werten, wie der Querzugsfestigkeit, einer nach Verpressen der beleimten Holzpartikel hergestellten Holzwerkstoffplatte zu setzen; d.h. es besteht eine Korrelation zwischen gemessener Bindemittelmenge und Querzugsfestigkeit. So bedingt eine schlechte Verteilung (angezeigt durch eine starke Schwankung) der gemessenen Bindemittelmenge eine reduzierte Querzugsfestigkeit und eine größere Schwankung in der Querzugsfestigkeit der verpressten Holzwerkstoffplatten.

Eine größere Schwankung der Querzugsfestigkeit erfordert demnach eine höhere Dosierung an Bindemittel um die normativ vorgegebenen Werte der Querzugsfestigkeit für Holzwerkstoffplatten, wie OSB oder Holzspanplatten zu erreichen. Die Querzugsfestigkeit von OSB sollte typischerweise in einem Bereich zwischen 0,3 und 0,6 N/mm², bevorzugt zwischen 0,4 und 0,6 N/mm², insbesondere bevorzugt zwischen 0,45 und 0,55 N/mm² liegen.

Selbstverständlich können die aufgenommenen NIR-Spektren auch zur Analyse von weiteren Parametern der Holzpartikel, insbesondere Größenverteilung der Holzpartikel, Feuchtegehalt der Holzpartikel, verwendet werden.

Erfindungsgemäß wird als Bindemittel ein Isocyanat-haltiges Bindemittel, bevorzugt auf der Basis von aromatischen Polyisocyanaten, insbesondere Polydiphenylmethandiisocyanat (PMDI), Toluylendiisocyanat (TDI) und/oder Diphenylmethandiisocyanat (MDI) verwendet, wobei PMDI besonders bevorzugt ist.

Die Menge an auf die Holzpartikel aufgetragenem Bindemittel beträgt in einer Ausführungsform zwischen 2,0 und 3,5 Gew%, bevorzugt zwischen 2,5 und 3,5 Gew%, insbesondere bevorzugt zwischen 2,5 und 3,0 Gew% bezogen auf die Holzstrands.

Im Folgenden werden zwei Arten von Holzwerkstoffplatten und deren Herstellungsverfahren beschrieben, in denen das erfindungsgemäße Messverfahren einsetzbar ist.

Wie bereits erwähnt, werden mit dem vorliegenden Verfahren erfindungsgemäß Holzstrands verwendet. Es können auch Holzspäne, z.B. Recyclingspäne, als Holzpartikel verwendet werden. Holzstrands werden zur Herstellung von OSB und Holzspäne zur Herstellung von Holzspanplatten verwendet.

Die vorliegend verwendeten Holzstrands können eine Länge zwischen 50 bis 200 mm, bevorzugt 70 bis 180 mm, insbesondere bevorzugt 90 bis 150 mm; eine Breite zwischen 5 bis 50 mm, bevorzugt 10 bis 30 mm, insbesondere bevorzugt 15 bis 20 mm; und eine Dicke zwischen 0,1 und 2 mm, bevorzugt zwischen 0, 3 und 1,5 mm, insbesondere bevorzugt zwischen 0, 4 und 1 mm aufweisen. In einer Ausführungsform weisen die Holzstrands z.B. eine Länge zwischen 150 und 200 mm, eine Breite zwischen 15 und 20 mm, eine Dicke zwischen 0,5 und 1 mm und eine Feuchte von max. 50% auf.

Wie erwähnt, werden die Strands zur Herstellung von OSB verwendet. Die Herstellung der OSB erfolgt in einem mehrstufigen Prozess, wobei zunächst die Strands aus entrindetem Rundholz, bevorzugt Nadelhölzer, in Längsrichtung durch rotierende Messer abgeschält werden. Im sich anschließenden Trocknungsvorgang wird die natürliche Feuchtigkeit der Strands bei hohen Temperaturen reduziert. Der Feuchtigkeitsgrad der Strands kann je nach verwendeten Klebstoff variieren, wobei sich die Feuchtigkeit deutlich unter 10% bewegen sollte, um Spalter durch zu hohen Dampfdruck in der Platte beim späteren Verpressen zu vermeiden. In Abhängigkeit vom Klebstoff kann eine Benetzung auf eher feuchten Strands oder auf trockenen Strands günstiger sein.

Im Anschluss an die Trocknung der Strands werden diese in eine Beleimvorrichtung eingeführt, in welcher der Leim bzw. Klebstoff fein verteilt auf die Strands aufgebracht wird.

Eine für das vorliegende Verfahren verwendete Beleimungseinrichtung in Form eines Coils besteht aus einem rotierenden Zylinder und mindestens einem Versorgungsbalken für die Zufuhr von Klebstoff und Druckluft mit Sprühtellern, für deren Antrieb in Gehäusen angeordnete pneumatische Antriebsmotoren vorgesehen sind. Ein derartige Beleimungseinrichtung ist in der DE 10 2008 046 637 A1 beschreiben.

Ein geeigneter Trogmischer wird z.B. von der Firma Dieffenbacher vertrieben. Derartige Leimmischer sorgen durch ihr großes Kammervolumen und die optimale Verweilzeit des Materials für eine gleichmäßige Leimverteilung. Um ein optimales Beleimergebnis zu gewährleisten, wird der konstante Füllstand des Mischers durch eine Auslassklappe aufrechterhalten.

Nach der Beleimung werden die beleimten Strands in Streuapparaturen alternierend längs und quer zur Produktionsrichtung gestreut, so dass die Strands kreuzweise in mindestens drei Schichten angeordnet sind (untere Deckschicht - Mittelschicht - obere Deckschicht). Die Streurichtung von unterer und oberer Deckschicht ist dabei gleich, weichen jedoch von der Streurichtung der Mittelschicht ab. Auch unterscheiden sich die in der Deckschicht und Mittelschicht verwendeten Strands voneinander. So sind die in den Deckschichten verwendeten Strands flächig und die in der Mittelschicht verwendeten Strands weniger flächig bis hin zu spanförmig. Die Strands in der Mittelschicht können qualitativ schlechter sein, da die Biegefestigkeit im Wesentlichen durch die Deckschichten erzeugt wird. Deshalb kann auch Feingut, das beim Zerspanen entsteht, in der Mittelschicht von OSB-Platten verwendet werden. Im Anschluss an die Streuung der Strands erfolgt ein kontinuierliches Verpressen der selbigen unter hohem Druck und hoher Temperatur von z.B. 200 bis 250°C.

Geeigneterweise wird die NIR-Messung des vorliegenden Verfahrens zur Bestimmung der Menge und Verteilung des Bindemittels auf den Strands nach der letzten Streuvorrichtung durchgeführt, d.h. der NIR-Messkopf ist nach der letzten Streuvorrichtung angeordnet. Es ist aber auch denkbar, dass NIR-Messungen jeweils nach Streuen der Einzelschichten (d.h. untere Deckschicht-Mittelschicht-obere Deckschicht) erfolgen; d.h. jeweils ein NIR-Messkopf ist nach der ersten, zweiten und dritten Streuvorrichtung angeordnet.

Die mit hergestellten Grobspanplatten (OSB) weisen eine Querzugsfestigkeit von größer als 0,3 N/mm², bevorzugt von größer 0,4 N/mm², bevorzugt von größer 0,45 N/mm², insbesondere bevorzugt größer 0,5 N/mm² auf. Die Querzugsfestigkeit kann in einem Bereich zwischen 0,3 und 0,6 N/mm², bevorzugt zwischen 0,4 und 0,6 N/mm², insbesondere bevorzugt zwischen 0,45 und 0,55 N/mm² liegen.

Des Weiteren weisen Grobspanplatten (OSB) E-Module in Längsrichtung zwischen 3200 und 4000 MPa, bevorzugt zwischen 3400 und 3800 MPa und E-Module in Querrichtung zwischen 1400 und 1800 MPa, bevorzugt zwischen 1500 und 1600 MPa auf.

Die vorliegende OSB-Holzwerkstoffplatte kann eine Rohdichte zwischen 300 und 1000 kg/m³, bevorzugt zwischen 500 und 800 kg/m³, insbesondere bevorzugt zwischen 600 und 700 kg/m³ aufweisen.

Die Dicke der vorliegenden OSB-Holzwerkstoffplatte kann zwischen 5 und 50 mm, bevorzugt zwischen 8 und 40 mm betragen, wobei insbesondere eine Dicke zwischen 8 und 30 mm bevorzugt ist.

Wie erwähnt, können auch beleimte Holzspäne, z.B. Recyclingspäne gewonnen aus Recyclingholz, mit dem vorliegenden Verfahren vermessen werden. Holzspäne werden in einem Zerspanungsprozess hergestellt und in feines und grobes Spanmaterial unterteilt, wobei die größeren Holzspäne bevorzugt in der Mittelschicht einer Spanplatte verwendet werden und die kleineren Holzspäne bevorzugt in den Deckschichten verwendet werden.

Nach dem Beleimen werden die beleimten Holzspäne auf ein Transportband unter Ausbildung eines Spankuchens aufgestreut. Im Falle der Holzspäne wird üblicherweise mit einer Windstreuung gearbeitet, wobei zunächst eine erste Deckschicht, gefolgt von der Mittelschicht und abschließend eine zweite Deckschicht gestreut wird.

Auch im Falle der Herstellung von Holzspanplatten erfolgt die NIR-Messung geeigneterweise nach der letzten Streuvorrichtung d.h. der NIR-Messkopf ist nach der letzten Streuvorrichtung angeordnet. Es ist aber auch denkbar, dass NIR-Messungen jeweils nach Streuen der Einzelschichten (d.h. untere Deckschicht-Mittelschicht-obere Deckschicht) erfolgen; d.h. jeweils ein NIR-Messkopf ist nach der ersten, zweiten und dritten Streuvorrichtung angeordnet

Der Spankuchen wird anschließend zunächst vorgepresst und anschließend bei Temperaturen zwischen 100°C und 250°C, bevorzugt 130°C und 220°C, insbesondere bei 200°C heiß verpresst.

In beiden Fällen (OSB, Holzspanplatten) sind die NIR-Messköpfe jeweils mit einem Steuerungssystem mit Auswerteeinheit und Datenbank zur Prozessierung und Speicherung der ermittelten NIR-Daten verbunden. Bei Abweichungen der gemessenen Ist-Werte von den Soll-Werten erfolgt eine automatische Anpassung durch die Anlagensteuerung bzw. - regelung. Grundsätzlich liefern alle in einer Produktionslinie verwendeten NIR-Messköpfe die von ihnen gemessenen Istwerte an die zentrale Steuer- und Auswerte-Einheit liefern, die bei Abweichung der gemessenen Ist-Werte z.B. eines einzigen der NIR-Messköpfe von den entsprechenden Sollvorgaben den Produktionsablauf entsprechend regelt oder vorausschauend steuert.

Es wird somit ein Verfahren bereitgestellt, in welchem durch die Verwendung eines NIR-Messkopfes, die Menge und Verteilung eines auf Holzpartikel aufgetragenen Bindemittels aus einem einzigen NIR-Spektrum bzw. der Reflektion bzw. Streuung von NIR-Strahlung bestimmt werden kann, und zwar durch eine berührungslose Messung. Die mit dem Messkopf oder den Messköpfen ermittelten Daten werden in einer vorteilhaften Ausprägung der Erfindung direkt zur Anlagensteuerung oder -regelung verwendet.

Zudem wird in einer weiteren vorteilhaften Ausprägung der Erfindung durch die Speicherung der Daten eine Verbesserung der Qualitätskontrolle ermöglicht. Auch bei Anlagenversuchen können die gespeicherten Daten vorteilhaft einen Beitrag zur Auswertung liefern, z.B. Inbetriebnahme einer Anlage bei Neuinstallation oder nach einer Wartung oder Reparatur oder zu in-situ Testzwecken von neuen Produktions- oder Messverfahren. Durch das sofortige Vorliegen der Messwerte und der hohen Messfrequenz wird eine sehr enge Kontrolle bzw. Steuerung oder Regelung der Anlagen ermöglicht.

Die Vorteile des vorliegenden Verfahrens sind vielfältig: Berührungslose Multiparameterbestimmung ("real time"- oder "Echtzeit"-Messung) mit deutlich reduzierter zeitlicher Verzögerung in der Auswertung der gemessenen Parameterwerte; verbesserte Anlagensteuerung bzw. -regelung, Verringerung des Ausschusses, Verbesserung der Qualität der auf der Anlage hergestellten Produkte, Kostenreduzierung und Verbesserung der Anlagenverfügbarkeit.

Das Steuerungssystem der jeweiligen Fertigungsanlage umfasst mindestens eine computergestützte Auswerteeinheit (bzw. Prozessoreinheit) und eine Datenbank. In der Auswerteeinheit erfolgt der Abgleich bzw. Vergleich des für das Produkt (d.h. beschichtetes Trägermaterial) gemessenen NIR-Spektrums mit den für die jeweils einzelnen Parameter erstellten Kalibriermodellen. Die so bestimmten Parameterdaten werden in der Datenbank gespeichert.

Die mit dem vorliegenden spektroskopischen Verfahren bestimmten Daten können zur Steuerung der jeweiligen Fertigungslinie verwendet werden. Die berührungslos gemessenen Parameterwerte des NIR-Multimesskopfes ("Ist-Werte") können, wie zuvor bereits beschrieben, direkt und in "real time" für die Steuerung bzw. Regelung der betreffenden Anlage verwendet werden, indem beispielsweise die gemessenen und in der Datenbank, z.B. einer relationalen Datenbank, Ist-Werte gespeichert und mit dort vorhandenen Soll-Werten dieser Parameter verglichen werden. Die sich ergebenden Differenzen werden anschließend zur Steuerung bzw. Regelung der Produktionslinie verwendet.

Für den Abgleich und die Steuerung der jeweiligen Fertigungslinie wird ein computerimplementiertes Verfahren sowie ein Computerprogramm umfassend Befehle, die bei der Ausführung des Programms durch einen Computer diesen veranlassen das computerimplementierte Verfahren auszuführen, bereitgestellt. Das Computerprogramm ist in einer Speichereinheit des Steuerungssystems der jeweiligen Fertigungslinie gespeichert.

Die Erfindung wird nachfolgend an Ausführungsbeispielen unter Bezugnahme auf die Figuren näher erläutert. Es zeigen:
- Figur 1: NIR-Spektren aufgenommen von mit PMDI besprühten Holzstrands mit einer Nullprobe; und
- Figur 2 A, B: die Verteilung vom PMDI auf Holzstrands.

### Ausführungsbeispiel 1: Erstellung Kalibrationsmodell

In einer drehbar gelagerten Trommel wurde mit einer Sprühvorrichtung PMDI in verschiedenen Konzentrationen auf Deckschicht-Strands (1, 3 und 5 Gew% auf Holz) aufgesprüht. Die Strands, die aus Kiefernholz hergestellt worden waren, waren nach der Trocknung aus einer industriellen Produktion entnommen worden. Während des Aufsprühens wurde die Trommel mit einer Kurbel händisch gedreht. Dies wurde für jede Konzentration fünfmal wiederholt.

Von den einzelnen Versuchen wurden Strands (5 Stück) entnommen und mit Hilfe eines NIR-Messgerätes der Fa. Perkin Elmer NIR-Spektren aufgenommen. Dabei werden pro entnommenem Strand fünf Einzelspektren erstellt. Zu diesem Zweck wird der Strand unter dem Messkopf bewegt. Die Einzelspektren wurden dann zu einem gemittelten Spektrum vereinigt. Dann wurde mit Hilfe einer Software ein Kalibrationsmodell erarbeitet. Bei der Software handelt es sich um eine multivariate Datenanalyse.

Dafür werden NIR-Spektren zwischen 700 und 2500 nm aufgenommen. Insbesondere der Bereich zwischen 1350 und 2500 nm wird für die Erstellung des Kalibrationsmodells genutzt. Eine Nullprobe ohne PMDI wurde ebenfalls analysiert (siehe).

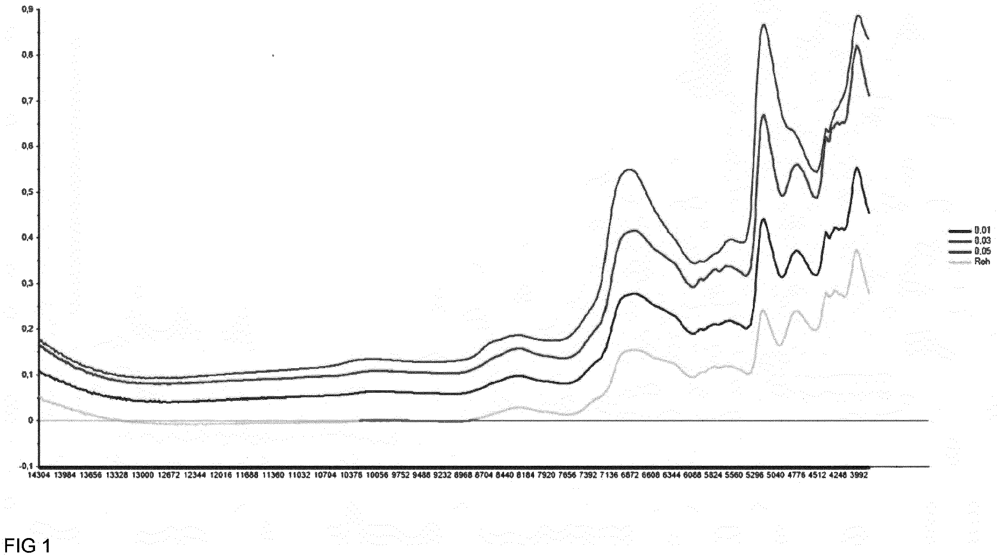

Die im Diagramm der Figur 1 gezeigten NIR-Spektren zeigen eine gute Korrelation zwischen der Menge an aufgetragenen PMDI (0, 1, 3, 5 Gew%) und der Messsignalstärke.

### Ausführungsbeispiel 2: Bestimmung PMDI-Gehalt an Mittelschichtstrands

An einer OSB-Linie wurden nach der Beleimung in einem Trogmischer Mittelschicht-Strands entnommen und auf ihren PMDI-Gehalt geprüft. Der Sollwert sollte laut Rezeptierung bei 2,9 Gew% liegen. Bei den zehn entnommenen Proben wurden mit einem NIR-Messkopf Spektren erstellt und mit Hilfe des Kalibrationsmodells der PMDI-Gehalt bestimmt. Er lag zwischen 2,6 und 3,2 Gew%.

### Ausführungsbeispiel 3: Bestimmung des PMDI-Gehalts bei Recyclingspänen

An einer OSB-Linie wurden in der Mittelschicht Recyclingspäne aus Recyclingholz verwendet. Diese Späne unterschieden sich farblich deutlich von den aus Waldholz hergestellten Deckschichtstrands. Sie waren zum Teil grau, bräunlich oder rötlich gefärbt. Diese Strands wurden im Trogmischer mit 3,5 Gew% PMDI beleimt. Es wurden Proben entnommen und diese mit dem NIR-Messkopf analysiert. Dabei wurden unterschiedlich gefärbte Proben erzeugt (Aussortieren).Trotz der Verfärbungen konnten Werte zwischen 3,1 und 3,6 Gew% bestimmt werden. Signifikante Ausreiser wurden nicht beobachtet.

### Ausführungsbeispiel 4: Bestimmung PMDI-Gehalt von OSB-Strands an einer Produktionslinie zur Herstellung von OSB

Ein NIR-Messkopf wurde mittig über einer OSB-Linie kurz vor der Contipresse installiert. Während der Produktion wurden kontinuierlich NIR-Spektren der mit PMDI beleimten Strands aufgenommen. Es wurden acht Spektren pro Minute erstellt. Die Spektren wurden über eine Software als ein Mittelwert über eine bestimmte Anzahl von Spektren dargestellt. Der PMDI-Gehalt von 2,9 Gew% wurde im Mittel bestätigt. Dann wurde die Beleimung von 2,9 Gew% auf 3,5 Gew% angehoben. Diese Anhebung wurde mit Hilfe des NIR-Messkopfes nachvollzogen.

Mit Hilfe von unbeleimten Strands wurde eine ungleichmäßige Beleimung simuliert. Während einer Produktion in der die Deckschichtstrands mit 2,9 Gew% PMDI beleimt waren, wurden in regelmäßigen Zeitabständen unbeleimte Strands unmittelbar vor dem Messkopf auf den Strandkuchen geworfen. Es zeigte sich, dass der Mittelwert des Messsignals langsam absank und damit einen niedrigeren mittleren Leimanteil anzeigte. Zudem war auch eine stärkere Schwankung des Messsignals zu beobachten.

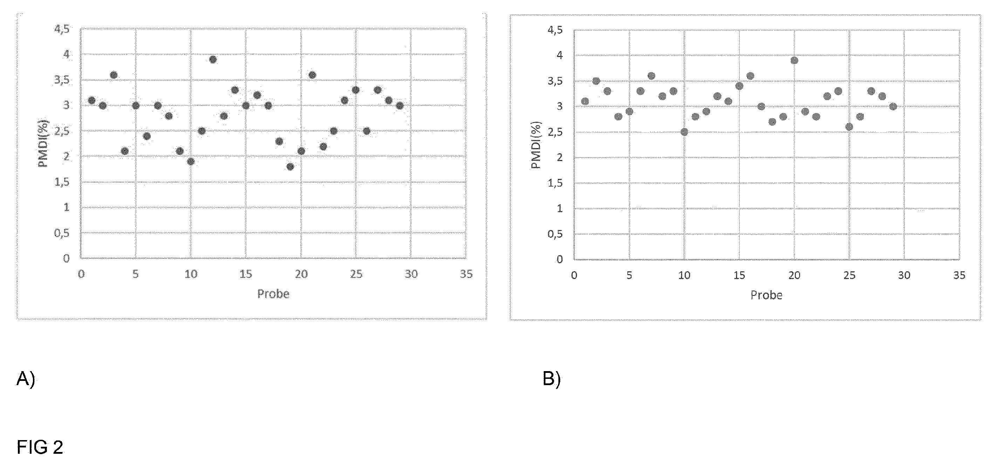

Die Schwankung des Messsignals in Abhängigkeit der Bindemittelverteilung auf den Holzpartikeln ist beispielhaft in den Figuren 2 A,B dargestellt.

So zeigt das Diagramm der Figur 2A eine schlechte Verteilung des Bindemittels. In dem Beispiel der Fig. 2A liegt die durchschnittlich gemessene Bindemittelmenge bei 2,8 Gew%. Die gemessenen Werte schwanken um diesen Mittelwert zwischen 35,7% (größte Abweichung nach unten bei 1,8 Gew%) und 39,3 % (größte Abweichung nach oben bei 3,9 Gew%).

Das Diagramm der Figur 2B zeigt hingegen eine gute Verteilung des Bindemittels. In dem Beispiel der Fig. 2B liegt die durchschnittlich gemessene Bindemittelmenge bei 3,1 Gew%. Die gemessenen Werte schwanken um diesen Mittelwert zwischen 19,3% (größte Abweichung nach unten bei 2,5 Gew%) und 25,8% (größte Abweichung nach oben bei 3,9 Gew%).

Die durch eine on-line Messung erhaltenen Daten können archiviert werden, um bei später auftretenden Problemen bei den Produkten nachvollziehen zu können, ob Regelabweichungen vorhanden waren. Natürlich kann auch in Kombination mit der Bestimmung der technologischen Werte eine Steuerung der Leimdosierung erfolgen. Von besonderem Interesse ist natürlich auch, der Vergleich unterschiedlicher Leime im Hinblick auf deren Leistungsfähigkeit. Durch die beschriebenen Analysen kann die Produktionslinie im Hinblick auf Kosten und Produktivität besser optimiert werden.

Es ergeben sich Vorteile:
- In-Line Analyse
- Bessere Prozessoptimierung möglich
- Analyse der Leimverteilung
- Einfachere Fehleranalyse
- Kostenreduzierung

## Patentansprüche

1. Messverfahren zur online Bestimmung einer auf Holzstrands aufgetragenen Menge und Verteilung eines Isocyanat-haltigen Bindemittels in einer Produktionsanlage zur Herstellung von Holzwerkstoffplatten,
umfassend die Schritte
- Aufbringen von mindestens einem Isocyanathaltigen Bindemittel in jeweils unterschiedlichen quantitativ definierten Mengen auf jeweilige Proben aus Holzstrands als Referenzproben und Aufstreuen der Referenzproben auf ein Transportband;
- Aufnahme von mindestens einem NIR-Spektrum jeder der auf das Transportband aufgestreuten Referenzproben unter Verwendung von mindestens einem NIR-Messkopf in einem Wellenlängenbereich zwischen 1800 nm und 2500 nm,
- Zuordnung der unterschiedlichen quantitativen Mengen an Isocyanathaltigen Bindemittel der auf das Transportband aufgestreuten Referenzproben zu den aufgenommenen NIR-Spektren der genannten Referenzproben; und
- Erstellung eines Kalibriermodells für den Zusammenhang zwischen den spektralen Daten der NIR-Spektren und den dazugehörigen quantitativen Mengen an Isocyanathaltigen Bindemittel der auf das Transportband aufgestreuten Referenzproben mittels einer multivariaten Datenanalyse;
- Aufbringen von mindestens einem IsocyanathaltigenBindemittel auf Holzstrands und Aufstreuen der mit dem Isocyanathaltigen Bindemittel versehenen Holzstrands auf ein Transportband,
- Aufnehmen von mindestens einem NIR-Spektrum der mit dem IsocyanathaltigenBindemittel versehenen und auf das Transportband aufgestreuten Holzstrands unter Verwendung des mindestens einen NIR-Messkopfes in einem Wellenlängenbereich zwischen 1800 nm und 2500 nm, und
- Bestimmen der quantitativen Menge des auf die Holzstrands aufgebrachten Isocyanathaltigen Bindemittels durch Vergleich des für die mit dem Bindemittel versehenen Holzstrands aufgenommenen NIR-Spektrums mit dem erstellten Kalibriermodell,
wobei die Menge an auf die Holzstrands aufgetragenem Isocyanathaltigen Bindemittel zwischen 1,0 und 3,5 Gew% beträgt.

2. Verfahren nach Anspruch 1, **dadurch gekennzeichnet, dass** das Verfahren in einer Produktionsanlage mit einer Anlagengeschwindigkeit zwischen 200 und 500 mm/sec, bevorzugt zwischen 300 und 400 mm/sec durchgeführt wird.

3. Verfahren nach einem der vorhergehenden Ansprüche, **dadurch gekennzeichnet, dass** der mindestens eine NIR-Messkopf sich quer zur Laufrichtung des mit den beleimten Holzstrands bestreuten Transportbandes bewegt und über die gesamte Breite des Transportbandes traversiert.

4. Verfahren nach einem der vorhergehenden Ansprüche, **dadurch gekennzeichnet, dass** der mindestens eine NIR-Messkopf mehrere Spektren pro Minute, bevorzugt bis zu acht Spektren pro Minute aufnimmt, aus denen ein Mittelwert über die Anzahl der aufgenommenen Spektren gebildet wird.

5. Verfahren nach einem der vorhergehenden Ansprüche **dadurch gekennzeichnet, dass** die mittels NIR-Spektroskopie bestimmte Bindemittelmenge in Bezug zu den technologischen Werten, bevorzugt Querzugsfestigkeit, einer nach Verpressen der beleimten Holzstrands hergestellten Holzwerkstoffplatte gesetzt werden.

6. Verfahren nach einem der vorhergehenden Ansprüche, **dadurch gekennzeichnet, dass** für die Bestimmung der aufgebrachten Menge an Bindemittel spektrale Daten aus dem gesamten aufgenommenen Spektralbereich verwendet werden.

7. Verfahren nach einem der vorhergehenden Ansprüche, **dadurch gekennzeichnet, dass** ein Isocyanat-haltige Bindemittel auf der Basis von aromatischen Polyisocyanaten, insbesondere Polydiphenylmethandiisocyanat (PMDI), Toluylendiisocyanat (TDI) und/oder Diphenylmethandiisocyanat (MDI) verwendet wird, wobei PMDI besonders bevorzugt ist.

8. Verfahren nach einem der vorhergehenden Ansprüche, **dadurch gekennzeichnet, dass** die Menge an auf die Holzstrands aufgetragenem Isocyanathaltigen Bindemittel zwischen 2,0 und 3,5 Gew%, bevorzugt zwischen 2,5 und 3,5 Gew%, insbesondere bevorzugt zwischen 2,5 und 3,0 Gew% bezogen auf die Holzstrands beträgt.

9. Verfahren nach einem der vorhergehenden Ansprüche, **dadurch gekennzeichnet, dass** Holzstrands mit einer Länge zwischen 50 bis 200 mm, bevorzugt 70 bis 180 mm, insbesondere bevorzugt 90 bis 150 mm; einer Breite zwischen 5 bis 50 mm, bevorzugt 10 bis 30 mm, insbesondere bevorzugt 15 bis 20 mm; und einer Dicke zwischen 0,1 und 2 mm, bevorzugt zwischen 0, 3 und 1,5 mm, insbesondere bevorzugt zwischen 0, 4 und 1 mm verwendet werden.

10. Verfahren nach einem der vorhergehenden Ansprüche, **dadurch gekennzeichnet, dass** die aufgenommenen NIR-Spektren zur Analyse von weiteren Parametern der Holzstrands, insbesondere Größenverteilung der Holzstrands, Feuchtegehalt der Holzstrands, verwendet werden.

11. Verfahren nach einem der vorhergehenden Ansprüche, **dadurch gekennzeichnet, dass** die Bestimmung der auf die Holzstrands aufgebrachten Menge an Bindemittel in der Fertigungslinie nach dem Verlassen einer Vorrichtung zum Streuen der Holzstrands erfolgt.

## Claims

1. Measuring method for online determination of amount and distribution of an isocyanate-containing binder applied to wood strands in a production plant for the manufacture of wood-based panels,
comprising the steps
- Applying at least one isocyanate-containing binder in different quantitatively defined amounts to respective samples of wood strands as reference samples and scattering the reference samples on a conveyor belt;
- Recording at least one NIR spectrum of each of the reference samples scattered on the conveyor belt using at least one NIR measuring head in a wavelength range between 1800 nm and 2500 nm,
- Assigning the different quantitative amounts of isocyanate-containing binders of the reference samples scattered on the conveyor belt to the recorded NIR spectra of said reference samples; and
- Creating a calibration model for the relationship between the spectral data of the NIR spectra and the corresponding quantitative amounts of isocyanate-containing binders of the reference samples scattered on the conveyor belt by means of multivariate data analysis;
- Applying at least one isocyanate-containing binder to wood strands and scattering the wood strands provided with the isocyanate-containing binder onto a conveyor belt,
- Recording at least one NIR spectrum of the wood strands provided with the isocyanate-containing binder and scattered on the conveyor belt using the at least one NIR measuring head in a wavelength range between 1800 nm and 2500 nm, and
- Determining the quantitative amount of the isocyanate-containing binder applied to the wood strands by comparing the NIR spectrum recorded for the wood strands provided with the binder with the calibration model created,
wherein the amount of isocyanate-containing binder applied to the wood strands is between 1.0 and 3.5% by weight.

2. Method according to claim 1, **characterized in that** the method is carried out in a production plant at a plant speed of between 200 and 500 mm/sec, preferably between 300 and 400 mm/sec.

3. Method according to one of the preceding claims, **characterized in that** the at least one NIR measuring head moves transversely to the running direction of the conveyor belt scattered with the glued wood strips and traverses over the entire width of the conveyor belt.

4. Method according to one of the preceding claims, **characterized in that** the at least one NIR measuring head records several spectra per minute, preferably up to eight spectra per minute, from which an average value is formed over the number of recorded spectra.

5. Method according to one of the preceding claims, **characterized in that** the amount of binder determined by means of NIR spectroscopy is set in relation to the technological values, preferably transverse tensile strength, of a wood-based panel produced after pressing the glued wood strands.

6. Method according to one of the preceding claims, **characterized in that** spectral data from the entire recorded spectral range are used to determine the amount of binder applied.

7. Method according to one of the preceding claims, **characterized in that** an isocyanate-containing binder based on aromatic polyisocyanates, in particular polydiphenylmethane diisocyanate (PMDI), toluene diisocyanate (TDI) and/or diphenylmethane diisocyanate (MDI) is used, PMDI being particularly preferred.

8. Method according to one of the preceding claims, **characterized in that** the amount of isocyanate-containing binder applied to the wood strands is between 2.0 and 3.5% by weight, preferably between 2.5 and 3.5% by weight, more preferably between 2.5 and 3.0% by weight relative to the wood strands.

9. Method according to one of the preceding claims, **characterized in that** wood strands having a length of between 50 and 200 mm, preferably 70 to 180 mm, more preferably 90 to 150 mm; a width of between 5 and 50 mm, preferably 10 to 30 mm, more preferably 15 to 20 mm; and a thickness of between 0.1 and 2 mm, preferably between 0, 3 and 1.5 mm, more preferably between 0, 4 and 1 mm are used.

10. Method according to one of the preceding claims, **characterized in that** the recorded NIR spectra are used for the analysis of further parameters of the wood strands, in particular size distribution of the wood strands, moisture content of the wood strands.

11. Method according to one of the preceding claims, **characterized in that** the determination of the amount of binder applied to the wood strands in the production line takes place after leaving a device for scattering the wood strands.

## Revendications

1. Procédé de mesure pour la détermination en ligne d'une quantité appliquée sur des copeaux de bois et distribution d'un liant contenant des isocyanates dans une installation de production pour la fabrication de panneaux en matériaux dérivés du bois,
comprenant les étapes
- de dépôt d'au moins un liant contenant des isocyanates en respectivement différentes quantités définies quantitativement sur des échantillons respectifs de copeaux de bois comme échantillons de référence et de dispersion des échantillons de référence sur une bande transporteuse ;
- d'enregistrement d'au moins un spectre NIR de chacun des échantillons de référence dispersés sur la bande transporteuse à l'aide d'au moins une tête de mesure NIR dans une plage de longueurs d'onde comprise entre 1800 nm et 2500 nm,
- d'affectation des différentes quantités quantitatives de liant contenant des isocyanates des échantillons de référence dispersés sur la bande transporteuse aux spectres NIR enregistrés desdits échantillons de référence ; et
- de création d'un modèle d'étalonnage pour la corrélation entre les données spectrales des spectres NIR et les quantités quantitatives associées de liant contenant des isocyanates des échantillons de référence dispersés sur la bande transporteuse au moyen d'une analyse de données multivariée ;
- de dépôt d'au moins un liant contenant des isocyanates sur des copeaux de bois et de dispersion des copeaux de bois pourvus du liant contenant des isocyanates sur une bande transporteuse,
- d'enregistrement d'au moins un spectre NIR des copeaux de bois pourvus du liant contenant des isocyanates et dispersés sur la bande transporteuse à l'aide de l'au moins une tête de mesure NIR dans une plage de longueurs d'onde comprise entre 1800 nm et 2500 nm, et
- de détermination de la quantité quantitative du liant contenant des isocyanates déposé sur les copeaux de bois par comparaison du spectre NIR enregistré pour les copeaux de bois pourvus du liant avec le modèle d'étalonnage créé,
dans lequel la quantité de liant contenant des isocyanates appliqué sur les copeaux de bois est comprise entre 1,0 et 3,5 % en poids.

2. Procédé selon la revendication 1, **caractérisé en ce que** le procédé est réalisé dans une installation de production avec une vitesse d'installation comprise entre 200 et 500 mm/sec, de préférence entre 300 et 400 mm/sec.

3. Procédé selon l'une quelconque des revendications précédentes, **caractérisé en ce que** l'au moins une tête de mesure NIR se déplace transversalement par rapport au sens de marche de la bande transporteuse sur laquelle sont dispersés les copeaux de bois collés et traverse toute la largeur de la bande transporteuse.

4. Procédé selon l'une quelconque des revendications précédentes, **caractérisé en ce que** l'au moins une tête de mesure NIR enregistre plusieurs spectres par minute, de préférence jusqu'à huit spectres par minute, à partir desquels une valeur moyenne est formée sur le nombre de spectres enregistrés.

5. Procédé selon l'une quelconque des revendications précédentes, **caractérisé en ce que** la quantité de liant déterminée par spectroscopie NIR est fixée par rapport aux valeurs technologiques, de préférence la résistance à la traction transversale, d'un panneau en matériau dérivé du bois fabriqué après le compactage des copeaux de bois collés.

6. Procédé selon l'une quelconque des revendications précédentes, **caractérisé en ce que** des données spectrales de l'ensemble du domaine spectral enregistré sont utilisées pour la détermination de la quantité de liant déposée.

7. Procédé selon l'une quelconque des revendications précédentes, **caractérisé en ce qu'**un liant contenant des isocyanates est à base de polyisocyanates aromatiques, en particulier de polydiphénylméthandiisocyanate (PMDI), de diisocyanate de toluène (TDI) et/ou de diphénylméthandiisocyanate (MDI), dans lequel le PMDI est particulièrement préféré.

8. Procédé selon l'une quelconque des revendications précédentes, **caractérisé en ce que** la quantité de liant contenant des isocyanates appliqué sur les copeaux de bois est comprise entre 2,0 et 3,5 % en poids, de préférence entre 2,5 et 3,5 % en poids, de manière particulièrement préférée entre 2,5 et 3,0 % en poids par rapport aux copeaux de bois.

9. Procédé selon l'une quelconque des revendications précédentes, **caractérisé en ce que** des copeaux de bois avec une longueur comprise entre 50 et 200 mm, de préférence 70 et 180 mm, de manière particulièrement préférée 90 et 150 mm ; une largeur comprise entre 5 et 50 mm, de préférence 10 et 30 mm, de manière particulièrement préférée 15 et 20 mm ; et une épaisseur comprise entre 0,1 et 2 mm, de préférence entre 0,3 et 1,5 mm, de manière particulièrement préférée entre 0,4 et 1 mm sont utilisés.

10. Procédé selon l'une quelconque des revendications précédentes, **caractérisé en ce que** les spectres NIR enregistrés sont utilisés pour l'analyse d'autres paramètres des copeaux de bois, en particulier la distribution granulométrique des copeaux de bois, la teneur en humidité des copeaux de bois.

11. Procédé selon l'une quelconque des revendications précédentes, **caractérisé en ce que** la détermination de la quantité de liant déposée sur les copeaux de bois s'effectue dans la ligne de fabrication après avoir quitté un dispositif de dispersion des copeaux de bois.
